# EUROPEAN PATENT APPLICATION

(11) **EP 2 698 102 A1**
(43) Date of publication of application: **19.02.2014**
(21) Application number: 13180327.2
(22) Date of filing: 14.08.2013
(51) Int. Cl.: A61B 5/00, A61B 5/055, A61B 6/00, A61B 8/08, A61B 6/03

(54) **Multiple heterogeneous imaging systems for clinical and preclinical diagnosis**

(30) Priority: 15.08.2012 IL 22149212
(71) Applicant: Aspect Imaging Ltd., 60850 Shoham (IL)
(72) Inventor: Batt, Aryeh, 90440 Har Hevron (IL); Rapoport, Uri, 73115 Moshav Ben Shemen (IL)
(74) Representative: Lecomte & Partners

(57) **Abstract**

The present invention provides an MRI imaging system for generating a rendered image, comprising at least one MRI device adapted to image an object and an image processor; at least one first type photon detector to generate at least one first optical image of said object and at least one second type photon detector to generate at least one second optical image of said object; wherein said image processor is adapted to superimpose said MRI image, said first optical image and said second optical image of said object thereby generating a rendered 3D computer MRI image of said object.

## Description

### FIELD OF THE INVENTION

The present invention generally pertains to multiple heterogeneous imaging systems for clinical and preclinical diagnosis.

### BACKGROUND OF THE INVENTION

Multi-modality combinations of Single-Photon Emission Computed Tomography (SPECT) with Computed Tomography (CT) and Positron Emission Tomography (PET) with CT have proven to be highly successful in the clinic, and small animal SPECT/CT and PET/CT are becoming the norm in the research and drug development setting. However, the use of ionizing radiation from a high-resolution CT scanner is undesirable in any setting and particularly in small animal imaging, in laboratory experiments where it can result in radiation doses of sufficient magnitude that the experimental results can be influenced by the organism's response to radiation. The alternative use of magnetic resonance (MR) offers a high-resolution, non-ionizing method for anatomical imaging of laboratory animals. MR brings considerably more than its 3D anatomical capability, especially regarding the imaging of laboratory animals. Dynamic MR imaging techniques can facilitate studies of perfusion, oxygenation, and diffusion amongst others. Further, MR spectroscopy can provide images that can be related to the concentration of endogenous molecules in vivo. MR imaging of injected contrast agents extends MR into the domain of molecular imaging. MR can be combined with other techniques to increase the information available to the clinician or researcher.

For example, in combination with the nuclear medicine SPECT and PET modalities in small animal imaging, MR facilitates studies of dynamic processes such as biodistribution, pharmacokinetics, and pharmacodynamics.

US patent application US 2011/0270078 to Wagenaar et al. discloses a multi-modal imaging system for imaging of an object under study that includes a magnetic resonance imaging (MRI) apparatus and an MRI-compatible single-photon nuclear imaging apparatus imbedded within the RF coil of the MRI system such that sequential or simultaneous imaging can be done with the two modalities using the same support bed of the object under study during the imaging session. However, US patent application US 2011/0270078 discloses only a multi-modal system capable of combining MRI with SPECT or PET.

US patent US7831077 to Gering discloses a method for generating an image of a region of interest of a subject includes obtaining a set of magnetic resonance (MR) data for the region of interest. In addition, photographic data for at least one surface of the region of interest is obtained, for example, from a database or by using a camera for photographing the region of interest. An image is generated by using the set of MR data to generate a depiction of at least one structure of the region of interest and by using the photographic data to generate a depiction of at least one surface of the region of interest. However, in US patent US7831077 the data for the two modalities are not acquired simultaneously.

US patent application 2003/0179308 to Zamorano et al. discloses a system for generating an augmented reality image comprises a video camera for obtaining video data and a sensor for obtaining sensed data. An augmented reality processor is coupled to the video camera and to the sensor. The augmented reality processor is configured to receive the video data from the video camera and to receive the sensed data from the sensor and to receive other types of computed data, such as any imaging modality. A display device is coupled to the augmented reality processor. The augmented reality processor is further configured to generate for display on the display device a video image from the video data received from the video camera and to generate a corresponding data image from the sensed data received from the sensor or other computed data. The augmented reality processor is further configured to merge the video image and the corresponding data image generated from computed data or sensed data so as to generate the augmented reality image. The system employs a tracking system that tracks the position of the video camera. The system may also employ a robotic positioning device for positioning the video camera, and which may be coupled to the tracking system for providing precise position information. However, the MRI data are not acquired simultaneously with the video and other sensor data.

US patent application US 2005/0028482 to Cable et al. discloses systems and methods for multi-modal imaging with light and a second form of imaging. Light imaging involves the capture of low intensity light from a light-emitting object. A camera obtains a two-dimensional spatial distribution of the light emitted from the surface of the subject. Software operated by a computer in communication with the camera may then convert two-dimensional spatial distribution data from one or more images into a three-dimensional spatial representation. The second imaging mode may include any imaging technique that compliments light imaging. Examples include magnetic resonance imaging (MRI) and computer topography (CT). An object handling system moves the object to be imaged between the light imaging system and the second imaging system, and is configured to interface with each system. However, in US patent application US 2005/0028482 the object to be imaged in moved between the two imaging systems, so that the two images are not obtained simultaneously.

US patent application US 2010/0245543 to Greer et al. discloses systems and methods of using MR-compatible cameras to view magnetic resonance imaging procedures. The MR-compatible camera systems may include a casing with at least two openings, including one oriented to permit a camera to view a site, and another opening oriented to permit a light source to illuminate a portion of the site. The camera systems may be used with either closed bore or open bore MRI systems. However, US patent application US 2010/0245543 discloses a system only capable of providing illumination for a surgeon during a surgical procedure.]

The review by Lowry et al., see Molecular Fluorescence, Phosphorescence, and Chemiluminescence Spectrometry Analytical Chemistry, Vol. 80, No. 12, June 15, 4551-4574, 2008, is incorporated herein as a reference.

It is therefore a long felt need to provide a system and method for acquiring multi-modal images where acquisition of the images in the different modes is not separated in time, where the use of the images is not limited to aiding a surgeon during a surgical procedure, and where the second modality need not be SPECT or PET.

### SUMMARY OF THE INVENTION

It is an object of the present invention to disclose a system for to multiple heterogeneous imaging systems for clinical and preclinical diagnosis.

It is an object of the present invention to disclose an MRI imaging system for generating a rendered image. The system comprises, inter alia, at least one MRI device adapted to image an object and an image processor; at least one first type photon detector to generate at least one first optical image of the object and at least one second type photon detector to generate at least one second optical image of the object; wherein the image processor is adapted to superimpose the MRI image, the first optical image and the second optical image of the object thereby generating a rendered 3D computer MRI image of the object.

It is an object of the present invention to disclose an MRI imaging as defined above, wherein the at least one first type photon detector is selected from a group consisting of a CCD array, a camera, a photoconductive detector array, a photovoltaic detector array, a quantum dot array, a superconducting single-photon detector array, a photovoltaic cell array, a phototube array, and any combination thereof.

It is an object of the present invention to disclose an MRI imaging as defined above, wherein the at least one second type photon detector is selected from a group consisting of a CCD array, a camera, a photoconductive detector array, a photovoltaic detector array, a quantum dot array, a superconducting single-photon detector array, a photovoltaic cell array, a phototube array, and any combination thereof.

It is an object of the present invention to disclose an MRI imaging as defined above, wherein the image processor is adapted to render the image by a Boolean method of correlating or combining the at least one first and at least one second image features.

It is an object of the present invention to disclose an MRI imaging as defined above, wherein the Boolean method uses Boolean operators selected from the group consisting of OR, AND, NOT, EXCLUSIVE OR and any combination thereof.

It is an object of the present invention to disclose an MRI imaging as defined above, wherein the at least one first type or at least one second type photon detector is sensitive to at least one of a group of radiation ranges consisting of X-rays, far ultraviolet (UV), near UV, visible, near infrared (IR), far IR and any combination thereof.

It is an object of the present invention to disclose an MRI imaging as defined above, wherein the at least one first type or at least one second type photon detector is selected from a group consisting of X-ray, X-ray computed tomography (CT), positron emission tomography (PET), fluorescence and phosphorescence microscopy (FPM), far ultraviolet (UV) spectrometer, near UV spectrometer, visible spectrometer, near infrared (IR) spectrometer, far IR spectrometer, ultrasound (US) imaging, Raman spectrometer and any combination thereof.

It is an object of the present invention to disclose an MRI imaging as defined above, wherein the magnets in the MRI imaging system are selected from a group consisting of permanent magnets, superconducting magnets, and any combination thereof.

It is an object of the present invention to disclose an MRI imaging as defined above, wherein the at least one first type photon detector and at least one second type photon detector positions are deployed layered at an approximately uniform distance on the same geometric sphere from a volume of interest (VOI) within the MRI device, or assembled in an approximately multiform distance from the VOI within the MRI device.

It is an object of the present invention to disclose an MRI imaging as defined above, wherein the system comprises, inter alia, at least two MRI devices adapted to image an object: at least one first MRI scanning device operating at a first magnetic field intensity and generating a first plurality of MRI images of the object; and at least one second MRI scanning device operating at a second magnetic field intensity and generating a second plurality of MRI images of the object, and further wherein the image processor is configured to fuse the first plurality of MRI images and the second plurality of MRI images to generate a clear image representation of at least a portion of the object.

It is an object of the present invention to disclose an MRI imaging system for generating a rendered image with high SNR. This system comprises, inter alia, at least one scanning imaging device adapted to acquire either one or more 2D images or 3D images of a predefined portion of an object; at least one first type photon detector selected from a group consisting of FPM, far ultraviolet (UV) spectrometer, near UV spectrometer, visible spectrometer, near infrared (IR) spectrometer, far IR spectrometer, ultrasound (US) imaging, Raman spectrometer and any combination thereof, adapted to generate at least one first signal; the first detector is set to detect at least one first range of a spectrum selected from a group consisting of fluorescence, phosphorescence, and chemiluminescence spectrums; at least one second type photon detector selected from a group consisting of FPM, far UV spectrometer, near UV spectrometer, visible spectrometer, near IR spectrometer, far IR spectrometer, US imaging, Raman spectrometer and any combination thereof, adapted to generate at least one second signal; the second detector is set to detect at least one second range of a spectrum selected from a group consisting of fluorescence, phosphorescence, and chemiluminescence spectrums; an image processor in communication with the scanner, first detector and second detector. The image processor is adapted to superimpose the MRI image, the first optical image and the second optical image of the object thereby generating a rendered 3D computer MRI image of the object.

It is an object of the present invention to disclose an MRI imaging as defined above, wherein per the predefined portion of the object, the at least one first type photon detector is adapted to generate at time Tᵢ at least one first signal S¹_{Tl}; and at another time, Tₙ, at least one second signal S¹_{Tn} is generated thereby at ΔTₙ, ΔS¹_{Tn} or Voxel_{xyz} ΔS¹_{Tn} are acquired; and wherein the at least one second type photon detector is adapted to generate at time Tᵢ at least one first signal S²_{Tn}; and at another time, Tₙ, at least one second signal S²_{Tn} is generated, thereby at ΔTₙ, ΔS²_{Tn} or Voxel_{xyz} ΔS²_{Tn} are acquired; n and i are integer numbers equal or greater than I.

It is an object of the present invention to disclose an MRI imaging as defined above, wherein per a predefined Voxel_{xyz} of the object, the image processor is adapted to operate in a method comprising steps of (i) acquiring the Voxel_{xyz} ΔS¹_{Tn}, Voxel_{xyz} ΔS²¹_{Tn} to Voxel_{xyz} ΔSⁿ_{Tn}; (ii) comparing all values and define to each Voxels_{xyz} a Voxel_{xyz} ΔS^{m}_{Tn} of maximum value, and (iii) superimposing the MRI image with the Voxel_{xyz} ΔSⁿ_{Tn} thereby acquiring for the Voxel_{xyz} a rendered MRI image of maximum SNR.

It is an object of the present invention to disclose an MRI imaging as defined above, wherein the image processor is adapted to operate in a method which further comprising steps of (iv) acquiring for at least a portion of the object ΣVoxel _{xi-n, yi-n zi-n xyz} ΔS^{m}_{Tn}; thereby acquiring for the portion of the object a rendered MRI image of maximum SNR.

It is an object of the present invention to disclose a method for generating a rendered 3D computer MRI image of an object comprising steps of layered deploying or assembling at an approximate uniform distance, on either same or different geometric sphere, from a volume of interest (VOI) within at least one MRI device, at least one first type photon detector and at least one second type photon detector; providing an object within the VOI and concurrently acquiring at least one MRI image, at least one first optical image, and at least one second optical image of the object; superimposing, by means of an image processor, the MRI image, the first optical image and the second optical image of the object thereby generating a rendered MRI image of the object.

It is an object of the present invention to disclose a method as defined above, wherein the step of generating a rendered MRI image of the object comprises processing of one or more members of a group consisting of the object's geometry, the viewpoint, the object's texture, lighting, and shading information.

It is an object of the present invention to disclose a method as defined above, wherein the method comprises additional steps of sequentially superimposing a plurality of MRI images, a plurality of the first optical images and a plurality of the second optical images of the object thereby generating a time-resolved image.

It is an object of the present invention to disclose a method as defined above, wherein the method comprises additional steps of selecting the at least one first type photon detector from a group consisting of a CCD array, a camera, a photoconductive detector array, a photovoltaic detector array, a quantum dot array, a superconducting single-photon detector array, a photovoltaic cell array, a phototube array, and any combination thereof.

It is an object of the present invention to disclose a method as defined above, wherein the method comprises additional steps of selecting the at least one second type photon detector from a group consisting of a CCD array, a camera, a photoconductive detector array, a photovoltaic detector array, a quantum dot array, a superconducting single-photon detector array, a photovoltaic cell array, a phototube array, and any combination thereof.

It is an object of the present invention to disclose a method as defined above, wherein the method is provided useful for imaging at least one first and at least one second image features; comprising additional steps of adapting the image processor to render the image by a Boolean method of correlating or combining the at least one first and at least one second image features.

It is an object of the present invention to disclose a method as defined above, wherein the method comprises additional steps of using for the Boolean method Boolean operators selected from the group consisting of OR, AND, NOT, EXCLUSIVE OR and any combination thereof.

It is an object of the present invention to disclose a method as defined above, wherein the method comprises additional steps of providing the at least one first type or at least one second type photon detector to be sensitive to at least one of a group of radiation ranges consisting of X-rays, far ultraviolet (UV), near UV, visible, near infrared (IR), far IR and any combination thereof.

It is an object of the present invention to disclose a method as defined above, wherein the method comprises additional steps of selecting the at least one first type or at least one second type photon detector from a group consisting of X-ray, X-ray computed tomography (CT), positron emission tomography (PET), fluorescence and phosphorescence microscopy (FPM), far ultraviolet (UV) spectrometer, near UV spectrometer, visible spectrometer, near infrared (IR) spectrometer, far IR spectrometer, ultrasound (US) imaging, Raman spectrometer and any combination thereof.

It is an object of the present invention to disclose a method as defined above, wherein the method comprises additional step of providing at least two MRI devices for imaging an object: operating at least one first MRI scanning device at a first magnetic field intensity and generating a first plurality of MRI images of the object; and operating at least one second MRI scanning device at a second magnetic field intensity and generating a second plurality of MRI images of the object, and fusing, by means of the image processor the first plurality of MRI images and the second plurality of MRI images thereby generating a clear image representation of at least a portion of the object.

It is an object of the present invention to disclose a method as defined above, wherein the method comprises additional steps of selecting magnets in the MRI imaging system from a group consisting of permanent magnets, superconducting magnets, and any combination thereof.

It is an object of the present invention to disclose a method for generating a rendered 3D computer MRI image of an object with maximal SNR. This method comprises steps of layered deploying or assembling at an approximate uniform distance, on either same or different geometric sphere, from a volume of interest (VOI) within at least one MRI device, at least one scanning imaging device adapted to acquire either one or more 2D images or 3D images of a predefined portion of an object; at least one first type photon detector selected from a group consisting of FPM, far ultraviolet (UV) spectrometer, near UV spectrometer, visible spectrometer, near infrared (IR) spectrometer, far IR spectrometer, ultrasound (US) imaging, Raman spectrometer and any combination thereof, adapted to generate at least one first signal; the first detector is set to detect at least one first range of a spectrum selected from a group consisting of fluorescence, phosphorescence, and chemiluminescence spectrums; at least one second type photon detector selected from a group consisting of FPM, far UV spectrometer, near UV spectrometer, visible spectrometer, near IR spectrometer, far IR spectrometer, US imaging, Raman spectrometer and any combination thereof, adapted to generate at least one second signal; the second detector is set to detect at least one second range of a spectrum selected from a group consisting of fluorescence, phosphorescence, and chemiluminescence spectrums; and an image processor in communication with the scanner, first detector and second detector; and superimposing, by means of an image processor, the MRI image, the first optical image and the second optical image of the object thereby generating a rendered 3D computer MRI image of the object.

It is an object of the present invention to disclose a method as defined above, wherein the method further comprises steps of defining at least one portion of the object; by means of the at least one first type photon detector, generating at time Tᵢ at least one first signal S¹_{Tl}; and at another time, Tₙ, generating at least one second signal S¹_{Tn}; thereby acquiring at ΔTₙ, ΔS¹_{Tn} or Voxel_{xyz} ΔS¹_{Tn;} by means of the at least one second type photon detector, generating at time Tᵢ at least one first signal S²_{Tn}; and at another time, Tₙ, at least one second signal S²_{Tn}, thereby acquiring at ΔTₙ, ΔS²_{Tn} or Voxel_{xyz} ΔS²_{Tn}.

It is an object of the present invention to disclose the method as defined above, wherein the method further comprises steps of defining Voxel_{xyz} within the object; acquiring the Voxel_{xyz} ΔS¹_{Tn}, Voxel_{xyz} ΔS²_{Tn} to Voxel_{xyz} ΔSⁿ_{Tn}; comparing all values and defining for each Voxels_{xyz} a Voxel_{xyz} ΔS^{m}_{Tn} of maximum value;, and superimposing the MRI image with the Voxel_{xyz} ΔSⁿ_{Tn} thereby acquiring for the Voxel_{xyz} a rendered MRI image of maximum SNR.

It is an object of the present invention to disclose a method as defined above, wherein the method further comprises steps of acquiring for at least a portion of the object ΣVoxel _{xi-n, yi-n, zi-n xyz} ΔS^{m}_{Tn}; thereby acquiring for the portion of the object a rendered MRI image of maximum SNR.

### BRIEF DESCRIPTION OF THE FIGURES

In order to better understand the invention and its implementation in practice, a plurality of embodiments will now be described, by way of non-limiting example only, with reference to the accompanying drawings, wherein

Fig. 1 schematically illustrates an animal subject inside a device of the present invention; and

Fig. 2 depicts a block diagram of operation of the system.

### DETAILED DESCRIPTION OF THE PREFERRED EMBODIEMNTS

The following description is provided, alongside all chapters of the present invention, so as to enable any person skilled in the art to make use of the invention and sets forth the best modes contemplated by the inventor of carrying out this invention. Various modifications, however, will remain apparent to those skilled in the art, since the generic principles of the present invention have been defined specifically to provide a means and method for determining the state of a process occurring in a reactor vessel using multi-modal sensing.

The term **'about'** hereinafter refers to ±20% of the defined measure.

The term **'substantially'** hereinafter refers to more than of about 90%.

The term **'plurality'** hereinafter refers to an integer greater than one.

It is in the scope of the invention wherein the term **'scanning imaging device'** refers hereinafter in a non limiting manner to an imaging device acquiring one or more 2D or 3D images, such as the one selected from a group consisting of X-ray computed tomography (CT), magnetic resonance imaging (MRI), positron emission tomography (PET), single-photon emission computed tomography (SPECT), fluorescence and phosphorescence microscopy (FPM) and any combination thereof.

Voxel (volumetric pixel or Volumetric Picture Element) is a volume element, representing a value on a regular grid in three dimensional space. Wikipedia says that voxel is analogous to a pixel, which represents 2D image data in a bitmap (which is sometimes referred to as a pixmap). As with pixels in a bitmap, voxels themselves do not typically have their position (their coordinates) explicitly encoded along with their values. Instead, the position of a voxel is inferred based upon its position relative to other voxels (i.e., its position in the data structure that makes up a single volumetric image). In contrast to pixels and voxels, points and polygons are often explicitly represented by the coordinates of their vertices. A direct consequence of this difference is that polygons are able to efficiently represent simple 3D structures with lots of empty or homogeneously filled space, while voxels are good at representing regularly sampled spaces that are non-homogeneously filled.

The system of the present invention provides a system of simultaneously acquiring MRI images and heterogeneous CCD images, where the CCD images can be, for non-limiting example, digital subtraction angiography images, high resolution cardiography images, low dose fluoroscopy images, digital radioagraphy images, or fluorescence images. The CCD arrays can be sensitive to, for non-limiting example, X-radiation, ultraviolet radiation, visible light, and infrared radiation.

In one embodiment of the system of the present invention, the system is used for preclinical or clinical examination of a living subject. For a non-limiting example, the MRI imaging system can be used to image the heart, while high resolution cardiography images can be used to image the blood vessels therein, thereby providing the clinician with images that enable the simultaneous observation of blood flow within and around the heart and the motion of the heart muscle.

In another example, a combination of MRI and radiography can be used to combine anatomical context and functional information, such as the anatomical delineation of the boundaries of a tumor (using, e.g., MRI) with the functional definition of aggressive cancer cells at the perimeter and necrotic cells at the core of the tumor (using, e.g. fluorescence images).

In yet another embodiment, MRI and near infrared spectroscopy (NIRS) in combination can be used for non-invasive assessment of brain function through the intact skull in human subjects, e.g., in branches of cognitive psychology, with NIRS detecting changes in blood hemoglobin concentrations associated with neural activity. This combination can also be used to detect tumors and other brain abnormalities, with MRI better adapted to identify the anatomical context of the tumor, and NIRS to identify the blood flow and/or neural activity accompanying the tumor or abnormality.

These embodiments are exemplary; many more uses and many more combinations will be obvious to one of ordinary skill in the art.

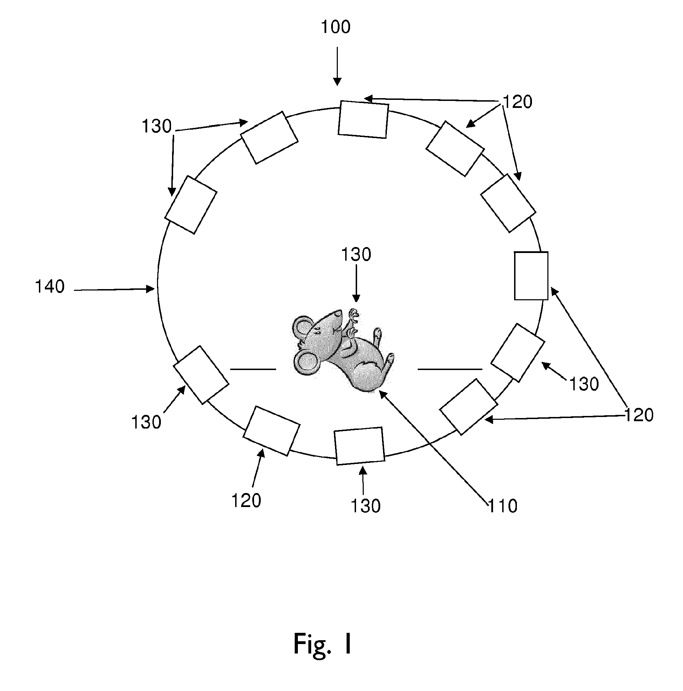

In reference to Fig. I, an embodiment is shown (100) in which a small mammal (110) is examined inside a chamber (140), the chamber comprising at least one MRI. Surrounding the animal (110) are sources (120) and detectors (130), shown illustratively in Fig. 1. These sources may comprise MRI RF probes, X-ray sources, far infrared sources, near infrared sources, visual light sources, near UV sources, far UV sources, ultrasound sources and any combination thereof. The subject is inside the field of field-generating sources such as MRI. Radiation from radiation sources passes through or is reflected from the subject. MRI receiver coils and CCD detectors are placed around the subject in a 2D or 3D arrangement to enable imaging of the desired region of the subject.

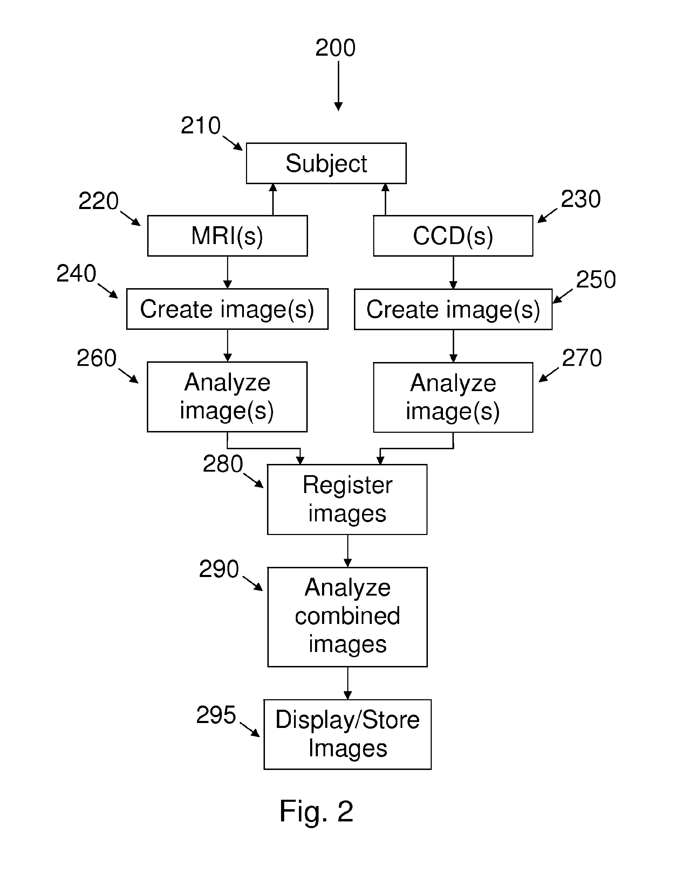

In reference to Fig. 2, a block diagram (200) is shown of the operation of the system. In Fig. 2, the subject (210) is scanned by at least one MRI (220) and at least one CCD (230). The signals from the at least one MRI scan are analyzed (240) to form at least one MRI image. Similarly, the signals from the at least one CCD are analyzed (230) to form at least one CCD image. The individual images can be analyzed (260, 270) to determine for non-limiting example, tumor shape and size, blood vessel distribution, or neural function. Images from different detectors can also be registered and aligned (280); subsequent combined analyses (290) provide the operator with simultaneously-acquired data on a desired region and can be used to determine, for non-limiting example, vascularity in tumors, distribution of aggressive and necrotic cells in tumors, and causes of blockage in blood vessels. Images, both individual and combined, can be displayed and can be stored for later use (295).

Registering and aligning techniques include rendering the images using Boolean methods of correlating and combining the images. Combining binary images using Boolean logic makes it possible to select structures or objects based on multiple criteria, such as, but not limited to, masking and threshholding. The Boolean operators commonly used are OR, AND, NOT, EXCLUSIVE OR and combinations thereof.

## Claims

1. An MRI imaging system for generating a rendered image, comprising at least one MRI device adapted to image an object and an image processor; at least one first type photon detector to generate at least one first optical image of said object and at least one second type photon detector to generate at least one second optical image of said object; wherein said image processor is adapted to superimpose said MRI image, said first optical image and said second optical image of said object thereby generating a rendered 3D computer MRI image of said object.

2. The system of claim 1, wherein at least one of the following is being held true (a) said at least one first type photon detector is selected from a group consisting of a CCD array, a camera, a photoconductive detector array, a photovoltaic detector array, a quantum dot array, a superconducting single-photon detector array, a photovoltaic cell array, a phototube array, and any combination thereof; (b) said at least one second type photon detector is selected from a group consisting of a CCD array, a camera, a photoconductive detector array, a photovoltaic detector array, a quantum dot array, a superconducting single-photon detector array, a photovoltaic cell array, a phototube array, and any combination thereof; and any combination thereof.

3. The system of claim 1, wherein said image processor is adapted to render said image by a Boolean method of correlating or combining said at least one first and at least one second image features.

4. The system of claim 3, wherein at least one of the following is being held true (a) said Boolean method uses Boolean operators selected from the group consisting of OR, AND, NOT, EXCLUSIVE OR and any combination thereof; (b) said at least one first type or at least one second type photon detector is sensitive to at least one of a group of radiation ranges consisting of X-rays, far ultraviolet (UV), near UV, visible, near infrared (IR), far IR and any combination thereof; (c) said at least one first type or at least one second type photon detector is selected from a group consisting of X-ray, X-ray computed tomography (CT), positron emission tomography (PET), fluorescence and phosphorescence microscopy (FPM), far ultraviolet (UV) spectrometer, near UV spectrometer, visible spectrometer, near infrared (IR) spectrometer, far IR spectrometer, ultrasound (US) imaging, Raman spectrometer and any combination thereof; (d) magnets in said MRI imaging system are selected from a group consisting of permanent magnets, superconducting magnets, and any combination thereof; and any combination thereof.

5. The MRI imaging system of claim 1 or any of its dependent claims, wherein said at least one first type photon detector and at least one second type photon detector are deployed layered at an approximately uniform distance on same geometric sphere from a volume of interest (VOI) within the MRI device, or assembled in an approximately multiform distance from the VOI within the MRI device.

6. The MRI imaging system of claim 1 or any of its dependent claims, comprising at least two MRI devices adapted to image an object: at least one first MRI scanning device operating at a first magnetic field intensity and generating a first plurality of MRI images of said object; and at least one second MRI scanning device operating at a second magnetic field intensity and generating a second plurality of MRI images of said object, and further wherein said image processor is configured to fuse said first plurality of MRI images and said second plurality of MRI images to generate a clear image representation of at least a portion of said object.

7. An MRI imaging system for generating a rendered image with high SNR, comprising:
a. at least one scanning imaging device adapted to acquire either one or more 2D images or 3D images of a predefined portion of an object;
b. at least one first type photon detector selected from a group consisting of FPM, far ultraviolet (UV) spectrometer, near UV spectrometer, visible spectrometer, near infrared (IR) spectrometer, far IR spectrometer, ultrasound (US) imaging, Raman spectrometer and any combination thereof, adapted to generate at least one first signal; said first detector is set to detect at least one first range of a spectrum selected from a group consisting of fluorescence, phosphorescence, and chemiluminescence spectrums;
c. at least one second type photon detector selected from a group consisting of FPM, far UV spectrometer, near UV spectrometer, visible spectrometer, near IR spectrometer, far IR spectrometer, US imaging, Raman spectrometer and any combination thereof, adapted to generate at least one second signal; said second detector is set to detect at least one second range of a spectrum selected from a group consisting of fluorescence, phosphorescence, and chemiluminescence spectrums;
d. an image processor in communication with said scanner, first detector and second detector;
wherein said image processor is adapted to superimpose said MRI image, said first optical image and said second optical image of said object thereby generating a rendered 3D computer MRI image of said object.

8. The MRI imaging system of claim 7, wherein per said predefined portion of said object, said at least one first type photon detector is adapted to generate at time Tᵢ at least one first signal S¹_{Tl}; and at another time, Tₙ, at least one second signal S¹_{Tn} is generated thereby at ΔTₙ, ΔS¹_{Tn} or Voxel_{xyz} ΔS¹_{Tn} are acquired; and wherein said at least one second type photon detector is adapted to generate at time Tᵢ at least one first signal S²_{Tn}; and at another time, Tₙ, at least one second signal S²_{Tn} is generated, thereby at ΔTₙ, ΔS²_{Tn} or Voxel_{xyz} ΔS²_{Tn} are acquired.

9. The MRI imaging system of claim 8, wherein per a predefined Voxel_{xyz} of said object, said image processor is adapted to operate in a method comprising steps of (i) acquiring said Voxel_{xyz} ΔS¹_{Tn}, Voxel_{xyz} ΔS²¹_{Tn} to Voxel_{xyz} ΔSⁿ_{Tn}; (ii) comparing all values and define to each Voxels_{xyz} a Voxel_{xyz} ΔS^{m}_{Tn} of maximum value, and (iii) superimposing said MRI image with said Voxel_{xyz} ΔSⁿ_{Tn} thereby acquiring for said Voxel_{xyz} a rendered MRI image of maximum SNR.

10. The MRI imaging system of claim 9, said image processor is adapted to operate in a method which further comprising steps of (iv) acquiring for at least a portion of said object ΣVoxel _{xi-n, yi-n, zi-n xyz} ΔS^{m}_{Tn}; thereby acquiring for said portion of said object a rendered MRI image of maximum SNR.

11. A method for generating a rendered 3D computer MRI image of an object comprising steps of layered deploying or assembling at an approximate uniform distance, on either same or different geometric sphere, from a volume of interest (VOI) within at least one MRI device, at least one first type photon detector and at least one second type photon detector; providing an object within said VOI and concurrently acquiring at least one MRI image, at least one first optical image, and at least one second optical image of said object; superimposing, by means of an image processor, said MRI image, said first optical image and said second optical image of said object thereby generating a rendered MRI image of said object.

12. The method of claim 11, wherein at least one of the following is being held true (a) said step of generating a rendered MRI image of said object comprising processing of one or more members of a group consisting object's geometry, viewpoint, object's texture, lighting, and shading information; (b) said method additionally comprising steps of sequentially superimposing a plurality of MRI images, a plurality of said first optical images and a plurality of said second optical images of said object thereby generating a time-resolved image; (c) comprising additional steps of selecting said at least one first type photon detector from a group consisting of a CCD array, a camera, a photoconductive detector array, a photovoltaic detector array, a quantum dot array, a superconducting single-photon detector array, a photovoltaic cell array, a phototube array, and any combination thereof; (d) said method comprising additional steps of selecting said at least one second type photon detector from a group consisting of a CCD array, a camera, a photoconductive detector array, a photovoltaic detector array, a quantum dot array, a superconducting single-photon detector array, a photovoltaic cell array, a phototube array, and any combination thereof; (e) said method comprising additional steps of providing said at least one first type or at least one second type photon detector to be sensitive to at least one of a group of radiation ranges consisting of X-rays, far ultraviolet (UV), near UV, visible, near infrared (IR), far IR and any combination thereof; and any combination thereof.

13. The method of claim 11 for imaging at least one first and at least one second image features; comprising additional steps of adapting said image processor to render said image by a Boolean method of correlating or combining said at least one first and at least one second image features.

14. The method of claim 13, comprising additional steps of using for said Boolean method Boolean operators selected from the group consisting of OR, AND, NOT, EXCLUSIVE OR and any combination thereof.

15. The method of claim 11, wherein at least one of the following is being held true (a) said method comprising additional steps of selecting said at least one first type or at least one second type photon detector from a group consisting of X-ray, X-ray computed tomography (CT), positron emission tomography (PET), fluorescence and phosphorescence microscopy (FPM), far ultraviolet (UV) spectrometer, near UV spectrometer, visible spectrometer, near infrared (IR) spectrometer, far IR spectrometer, ultrasound (US) imaging, Raman spectrometer and any combination thereof; (b) said method comprising additional step of providing at least two MRI devices for imaging an object: operating at least one first MRI scanning device at a first magnetic field intensity and generating a first plurality of MRI images of said object; and operating at least one second MRI scanning device at a second magnetic field intensity and generating a second plurality of MRI images of said object, and fusing, by means of said image processor said first plurality of MRI images and said second plurality of MRI images thereby generating a clear image representation of at least a portion of said object; (c) said method comprising additional steps of selecting magnets in said MRI imaging system from a group consisting of permanent magnets, superconducting magnets, and any combination thereof; and any combination thereof.

16. A method for generating a rendered 3D computer MRI image of an object with maximal SNR; said method comprising steps of
a. layered deploying or assembling at an approximate uniform distance, on either same or different geometric sphere, from a volume of interest (VOI) within at least one MRI device,
i. at least one scanning imaging device adapted to acquire either one or more 2D images or 3D image of a predefined portion of an object;
ii. at least one first type photon detector selected from a group consisting of FPM, far ultraviolet (UV) spectrometer, near UV spectrometer, visible spectrometer, near infrared (IR) spectrometer, far IR spectrometer, ultrasound (US) imaging, Raman spectrometer and any combination thereof, adapted to generate at least one first signal; said first detector is set to detect at least one first range of a spectrum selected from a group consisting of fluorescence, phosphorescence, and chemiluminescence spectrums;
iii. at least one second type photon detector selected from a group consisting of FPM, far UV spectrometer, near UV spectrometer, visible spectrometer, near IR spectrometer, far IR spectrometer, US imaging, Raman spectrometer and any combination thereof, adapted to generate at least one second signal; said second detector is set to detect at least one second range of a spectrum selected from a group consisting of fluorescence, phosphorescence, and chemiluminescence spectrums; and
iv. an image processor in communication with said scanner, first detector and second detector; and
b. superimposing, by means of an image processor, said MRI image, said first optical image and said second optical image of said object thereby generating a rendered 3D computer MRI image of said object.

17. The method of claim 16, wherein said method additional comprising steps of
a. defining at least one portion of said object;
b. by means of said at least one first type photon detector; generating at time Tᵢ at least one first signal S¹_{Tl}; and at another time, Tₙ, generating at least one second signal S¹_{Tn}; thereby acquiring at ΔTₙ, ΔS¹_{Tn} or Voxel_{xyz} ΔS¹_{Tn};
c. by means of said at least one second type photon detector, generating at time Tᵢ at least one first signal S²_{Tn}; and at another time, Tₙ, at least one second signal S²_{Tn} is generated, thereby acquiring at ΔTₙ, ΔS²_{Tn} or Voxel_{xyz} ΔS²_{Tn}.

18. The method of claim 17, wherein said method additionally comprises steps of
a. defining Voxel_{xyz} within said object;,
b. acquiring said Voxel_{xyz} ΔS¹_{Tn}, Voxel_{xyz} ΔS²_{Tn} to Voxel_{xyz} ΔSⁿ_{Tn};
c. comparing all values and define for each Voxels_{xyz} a Voxel_{xyz} ΔS^{m}_{Tn} of maximum value; and
d. superimposing said MRI image with said Voxel_{xyz} ΔSⁿ_{Tn} thereby acquiring for said Voxel_{xyz} a rendered MRI image of maximum SNR.

19. The method of claim 18, wherein said method additional comprising a step of
a. acquiring for at least a portion of said object ΣVoxel _{xi-n, yi-n, zi-n} ΔS^{m}_{Tn}; thereby acquiring for said portion of said object a rendered MRI image of maximum SNR.
